# EUROPEAN PATENT APPLICATION

(11) **EP 3 178 823 A1**
(43) Date of publication of application: **14.06.2017**
(21) Application number: 15198606.4
(22) Date of filing: 09.12.2015
(51) Int. Cl.: C07D 495/14, C09K 11/00, H01L 51/00, H05B 33/00

(54) **CHARGE TRANSPORTING MATERIAL FOR OPTOELECTRONIC AND/OR PHOTOELECTROCHEMICAL DEVICES**

(71) Applicant: Ecole Polytechnique Fédérale de Lausanne (EPFL), 1015 Lausanne (CH); Instituto Madrileno de Estudio Avanzados Nanocienca (IMDEA NANOCENCIA), 28049 Madrid (ES); Universidad Complutense de Madrid (UCM), 28040 Madrid (ES)
(72) Inventor: Nazeeruddin, MD. Khaja, 1024 Ecublens (CH); Graetzel, Michael, 1025 St-Sulpice (CH); Zimmermann, Iwan, 3123 Belp (CH); Roldan Carmona, Cristina, 1003 Lausanne (CH); Gratia, Paul, 1024 Ecublens (CH); Molina Ontoria, Augustín, 28007 Madrid (ES); Garcia Benito, Inés, 28095 Getafe Madrid (ES); Martín León, Nazario, 28038 Madrid (ES)
(74) Representative: Ganguillet, Cyril

(57) **Abstract**

The present invention relates to an optoelectronic and/or photoelectrochemical device comprising at least one organic-inorganic perovskite as sensitizer and at least one compound of formula (I) 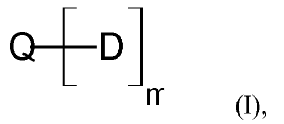 comprising one benzotrithiophene core Q, wherein each thiophene ring of said core is selected from a core according to any one of formulae (Ia), (Ib) and (Ic) 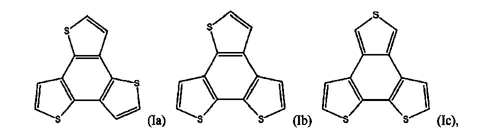 substituted by at least one heteroaromatic polycyclic system D being selected from a monocyclic system or a polycyclic system comprising fused aromatic rings or conjugated monocyclic aromatic rings comprising heteroatoms (O, S, Se and N) and being further substituted.

## Description

The present invention relates to charge transporting compound, to organic hole conductors and to hole transporting material comprising such a compound, to optoelectronic photoelectrochemical devices comprising such hole transporting material or hole transporting compound, in particular photovoltaic devices, organic-inorganic perovskite films or layer photovoltaic devices, p-n heterojunctions, dye-sensitized solar cells, organic solar cells and solid state solar cells.

### Prior Art and the Problem Underlying the Invention

The conversion of solar energy to electrical current using thin film third generation photovoltaics (PV) is being widely explored for the last two decades. The sandwich/monolithic-type PV devices, consisting of a mesoporous photoanode with an organic/inorganic light harvester, redox electrolyte/solid-state hole conductor, and counter electrode, have gained significant interest due to the ease of their fabrication, the flexibility in the selection of materials and the low cost effective production.

Perovskite based materials for optoelectronic applications were known in the literature since the pioneering work by Kagan et al. (C.R. Kagan, D. B. Mitzi, C. D. Dimitrakopoulos; Science, 1999, 286, 945). Since its first use as light absorber in a sensitized solar cell, organic-inorganic methylammonium (MA) lead halide MAPbX₃ (X = I, Br) perovskites have experienced a scientific research blast for photovoltaic applications. Organometal trihalide perovskites exhibit exceptional intrinsic properties, such as light absorption from visible to near-infrared range, high extinction coefficient, long electron-hole diffusion lengths, direct band gap, high charge carrier motilities, among others.

To obtain perovskite based photovoltaic devices showing high power conversion efficiencies (PCEs), the use of suitable electron- and hole-transporting materials (HTM) is of paramount importance in order to reduce charge recombination within the device and for an efficient charge extraction to the electrodes. The ambipolar behavior of the perovskite allows its combination with either electron transporting materials (ETMs) and/or with hole-transporting materials, which play an important role for transporting the charges. A wide number of HTMs have been synthesized and incorporated in photovoltaic solar cells (PSCs) ranging from classical semiconducting polymers to small molecules. Different central cores have been used, such as 9,9'-spirobifluorene, spiro-liked derivatives, thiophene derivatives, thiphenylamine, bridged-triphenylamines, pyrene, 3,4-ethylenedioxythiophene, linear π-conjugated structures: triptycene, tetraphenylethene silolothiophene or triazines, all of them namely decorated with diarylamines, triarylamines and/or carbazole derivatives. These materials act only as hole-transporters due to their limited absorption in the visible range.

There are fewer examples of HTMs absorbing in the visible and near-infrared region, which incorporate pentacene, *S,N*-heteropentacene, benzodithiophene derivatives, phenoxazine and thiolated nanographene, reaching PCEs over 15 %. To date, the best power conversion efficiency (PCE), up to 20%, was obtained using poly[bis(4-phenyl)(2,4,6-trimethylphenyl)amine] (PTAA) as HTM.

2,2,7,7-tetrakis(N,N-di-p-methoxyphenyl-amine)-9,9-Spirobifluorene (Spiro-OMeTAD) is the most studied HTM achieving PCEs over 19% by interface engineering of the devices, such as solid state solar cell using spiro-OMeTAD (2,2',7,7'-tetrakis(N,N-di-p-methoxyphenylamine) -9,9'-spirobifluorene) as organic hole transporting material (HTM) in combination with the hybrid organic-inorganic perovskite CH₃NH₃PbX₃, X being Cl-, Br or I⁻ (Snaith, H. J.; Moule, A. J.; Klein, C.; Meerholz, K.; Friend, R. H.; Gratzel, M. Nano Lett.; (Letter); 2007; 7(11)) and by controlling the formation of the perovskite via lewis base adduct. However, the preparation of the spirobifluorene central core requires an extensive synthetic protocols which make it relatively expensive. Besides, in order to achieve high PCEs devices, sublimation-grade of the Spiro-OMeTAD is an essential requirement.

Further, most of the polymers used as HTM present some problems related with the reproducibility, stemming from variable molecular weight and polydispersity index. On the contrary to small molecules, they have not very defined structures, complicate purification and characterization and therefore worse reproducibility.

Even the use of spiro-OMeTAD as HTM may trigger instability in solar cells and in particular solid state solar cells. Because Spiro-OMeTAD has two oxidation potentials being very close, this hole transporting material in the oxidized form is able to forms a di-cation, which in turn can dismutate and might cause device instability. Further, in many cases, the photocurrent is directly dependent on the yield in the hole transition from the absorber (oxidized perovskite or dye) to the solid p-conductor. This yield depends essentially on the thermodynamic driving force for the charge transfer, i.e. especially on the difference in the free enthalpy ΔG between dye and p-conductor. Since spiro-OMeTAD compound is present in semi-crystalline form, there is the risk that it will (re)crystallize in the processed form in the solar cell. In addition, the solubility in customary process solvents is relatively low, which leads to a correspondingly low degree of pore filling. Along stability issues, the high cost due to a complicated synthetic route and the high purity that is required (sublimation grade) in order to have good performance have been the main drawbacks for commercial applications of solid state solar cells.

The present invention addresses the disadvantage of organic hole transporting material, which provides instability to the device, when said hole transporter material is in oxidized form, as it is the case of spiro-OMeTAD.

The present invention also pursues to provide new hole transporting material, which does not required a step of sublimation during its purification after its synthesis as it is the case of the synthesis of spiro-OMeTAD and presents ease to be synthesized.

The present invention also pursues to provide new hole transporting material allowing to tune HOMO level and having positive impact on the sensitizer through its passivation to improve and provide higher power conversion efficiency (PCE) to photovoltaic devices comprising perovskite as well as to further optoelectronic devices Organic Light Emitting Diodes (OLED), Field effect Transistors (FET).

The present invention also pursues to provide new hole transporting material, which provides higher power conversion efficiency (PCE) to photovoltaic devices comprising perovskite as sensitizer or light absorbing material as well as to further optoelectronic devices Organic Light Emitting Diodes (OLED), Field effect Transistors (FET).

The present invention addresses the disadvantage due to the high prices of hole transporting materials and their expensive and complex synthesis. The synthesis process of hole transporting material involves expensive starting material compounds being not commercially available, very low temperature of reaction step, complexity, aggressive reagents and numerous steps (e.g. 5 steps for the Spiro-OMetTAD synthesis). Thus the synthesis process is lengthy, time-consuming and expensive and causes non-negligible environmental impact.

The invention pursues to provide an efficient solar cell, which can be rapidly prepared in an efficient way, using readily available or low cost materials such as conductive material, using a short manufacturing procedure based on industrially known manufacturing step, keeping the material costs and the material impact on the environment very low.

The present invention addresses the problems depicted above.

### Summary of the Invention

In an aspect, the present invention provides an optoelectronic and/or photoelectrochemical device comprising at least one organic-inorganic perovskite as sensitizer and at least one compound of formula (I) 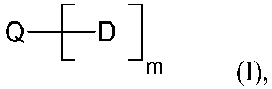 wherein
m is an integer selected from 1 to 4;
Q is a heteroaromatic polycyclic system comprising one benzotrithiophene core selected from a core according to any one of formulae (Ia), (Ib) and (Ic) 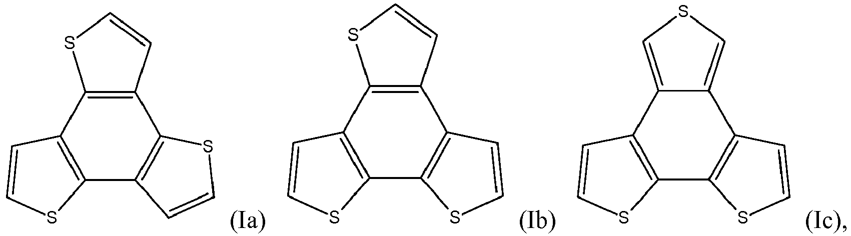 wherein
   at least one thiophene ring of said core is substituted by at least one heteroaromatic polycyclic system D;
D is selected from a polycyclic system of formula (1) 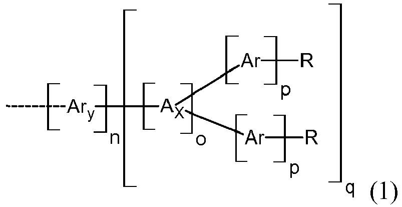 wherein,
   q is an integer selected from 1 or 2;
   o is an integer selected from 0 or 1;
   n and p are integers independently selected from 0, 1 or 2 with the proviso that, if n and o are 0, p is 1 or 2;
   A_{X} is selected from N or P(=O), preferably N;
   Ar_{y} and Ar are independently selected from a monocyclic system or a polycyclic system comprising fused aromatic rings or conjugated monocyclic aromatic rings, said ring comprising 0, 1 or 2 heteroatoms being selected from O, S, Se and N, and is further substituted in addition to R by other substituents independently selected from H, halogen (F, Cl, Br, I), C1-C10 alkyl, C1-C10 alkoxy group, C1-C10 alkylthio (-S-alkyl) and -C≡N;
   R is selected from H, R₁, -NR₁R₂, -O-R₁, -P(=O) R₁R₂, -S-R₁, or halogen (F, Cl, Br, I), wherein R₁ and R₂ are independently selected from C4-C20 aryl, C4-C20 heteroaryl, C4-C20 aryloxy group, C4-C20 heteroaryloxy group, C4-C20 alkoxyaryl, C4-C20 alkoxyheteroaryl, C4-C20 aryl aryloxy group, C4-C20 heteroaryl aryloxy group, C1-C20 alkyl, C1-C20 alkoxy group, C1-C20 alkoxyalkyl, C1-C20 alkylthio, C2-C20 alkenyl and C2-C20 alkynyl, wherein said alkyl, alkoxy, alkoxyalkyl, alkenyl and alkynyl, if they comprise 3 or more carbons, may be linear, branched or cyclic and wherein aryl, heteroaryl, alkyl, alkenyl, alkynyl may be further substituted by alkoxy group, alkylthio group and alkyl.

In a further aspect, the invention provides a p-n heterojunction comprising at least one organic-inorganic perovskite as sensitizer and at least one compound of the invention.

In another aspect, the invention provides the use of a compound of the invention to modulate the HOMO level.

Remarkably, in some aspects, the present inventors have found that a compound, which is based on benzotrithiophene (BTT) as central core being functionalized and substituted by further polycyclic system comprising fused aromatic or non-aromatic rings and/or conjugated monocyclic rings with at least one heteroatom and as being defined by a compound of formula (I) above, contributes to both effective charge extraction from perovskite absorber (Hole Transporting Material function) and photocurrent enhancement (passivation of the perovskite layer, good electron transmission performance and cavity transmission performance) in a optoelectronic and/or photoelectrochemical device, or a solid photovoltaic device. Said compound also improves the PCE of these devices, and in particular optoelectronic and/or photoelectrochemical device comprising perovskite pigment as sensitizer. The BTT core exhibits a planarized star-shaped structure which promotes an effective π-π intermolecular interaction and may be used as a new hole transporting material in said devices.

Although their large size, said compounds are good soluble in organic solvent, which greatly facilitates their purification and processing and their application or deposition on the sensitizer layer in the solid photovoltaic device. Moreover the synthesis process of these compounds involves less steps and the starting materials easily available.

Further aspects and preferred embodiments of the invention are detailed herein below and in the appended claims. Further features and advantages of the invention will become apparent to the skilled person from the description of the preferred embodiments given below.

### Brief Description of the Drawings

**Figure 1A** shows the scheme of the synthesis of the tribromo-benzotrithiophene (BTT) : a) I₂, H₅IO₆, sulfuric acid, rt, 98 %; b) Pd(PPh₃)₄, CuI, DIPA, THF, TMSA, reflux, 76 %; c) Na₂S, NMP, 185 °C, 42 %; d) NBS, chloroform:acetic acid (2:1), 60 °C, 81 %. **Figure 1B** shows the scheme of the synthesis of compound of formula (29) or BTT-1. **Figure 1C** shows the scheme of the synthesis of compound of formula (30) or BTT-2. **Figure 1D** shows the scheme of the synthesis of compound of formula (31) or BTT-3.
**Figure 2A** shows I-V curves showing the performance of BTT-1, BTT-2 and BTT-3 as hole transporting material in combination with MAPbI3 perovskite. **Figure 2B** shows I-V curves showing the performance of BTT-1, BTT-2 and BTT-3 on the compositional modification (FAPbI₃)_{0.95}(MAPBr₃)_{0.15}.
**Figure3** shows a semi-logarithmic plot of lateral conductivity of the doped BTT-1/BTT-2/BTT-3 molecules.

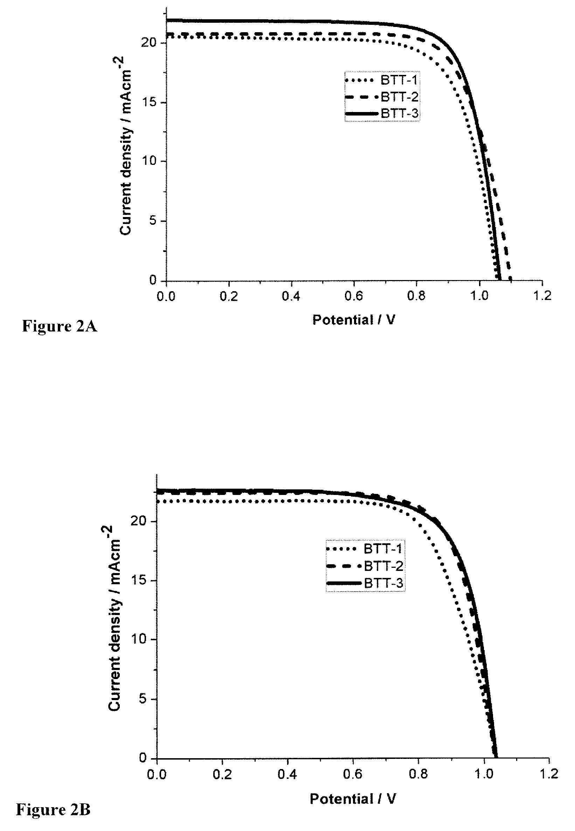

**Figure 2A** I-V curves showing the performance of bbb-BTT (compound of formula (32)) and bbc-BTT (compound of formula (33)) on MAPbI3 perovskite.

### Detailed Description of the Preferred Embodiments

The present invention concerns an optoelectronic and/or photoelectrochemical device, in particular a solid state solar cell, comprising at least one compound based on benzotrithiophene (BTT) as central core being functionalized and substituted by further polycyclic system comprising fused aromatic or non-aromatic rings and/or conjugated monocyclic rings with at least one heteroatom.

Specifically, the present invention concerns an optoelectronic and/or photoelectrochemical device comprising at least one organic-inorganic perovskite as sensitizer and at least one compound based on benzotrithiophene (BTT) as central core being functionalized and substituted by further polycyclic system comprising fused aromatic or non-aromatic rings and/or conjugated monocyclic rings with at least one heteroatom.

The invention concerns an optoelectronic and/or photoelectrochemical device comprising at least one organic-inorganic perovskite as sensitizer and at least one compound of formula (I) 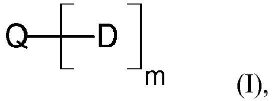 wherein
m is an integer selected from 1 to 4;
Q is a heteroaromatic polycyclic system comprising one benzotrithiophene core selected from a core according to any one of formulae (Ia), (Ib) and (Ic) 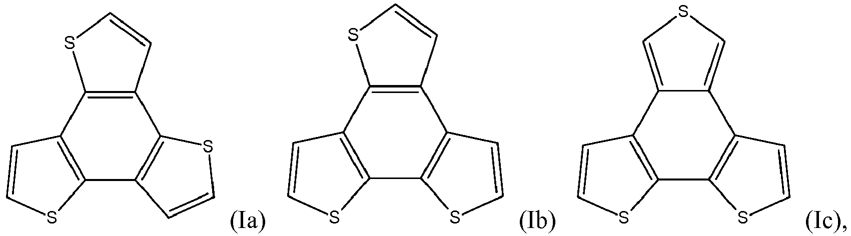 wherein
   at least one thiophene ring of said core is substituted by at least one
   heteroaromatic polycyclic system D;
D is selected from a polycyclic system of formula (1) 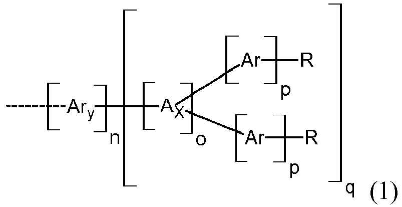 wherein,
   q is an integer selected from 1 or 2;
   o is an integer selected from 0 or 1;
   n and p are integers independently selected from 0, 1 or 2 with the proviso that, if n and o are 0, p is 1 or 2;
   A_{X} is selected from N or P(=O), preferably N;
   Ar_{y} and Ar are independently selected from a monocyclic system or a polycyclic system comprising fused aromatic rings or conjugated monocyclic aromatic rings, said ring comprising 0, 1 or 2 heteroatoms being selected from O, S, Se and N, and is further substituted in addition to R by other substituents independently selected from H, halogen (F, Cl, Br, I), C1-C10 alkyl, C1-C10 alkoxy group, C1-C10 alkylthio (-S-alkyl) and -C≡N;
   R is selected from H, R₁, -NR₁R₂, -O-R₁, -P(=O) R₁R₂, -S-R₁, or halogen (F, Cl, Br, I), wherein R₁ and R₂ are independently selected from C4-C20 aryl, C4-C20 heteroaryl, C4-C20 aryloxy group, C4-C20 heteroaryloxy group, C4-C20 alkoxyaryl, C4-C20 alkoxyheteroaryl, C4-C20 aryl aryloxy group, C4-C20 heteroaryl aryloxy group, C1-C20 alkyl, C1-C20 alkoxy group, C1-C20 alkoxyalkyl, C1-C20 alkylthio, C2-C20 alkenyl and C2-C20 alkynyl, wherein said alkyl, alkoxy, alkoxyalkyl, alkenyl and alkynyl, if they comprise 3 or more carbons, may be linear, branched or cyclic and wherein aryl, heteroaryl, alkyl, alkenyl, alkynyl may be further substituted by alkoxy group, alkylthio group and alkyl.

at least one thiophene ring of a benzotrithiophene core according to any one of formulae (Ia), (Ib) and (Ic)is substituted by at least one heteroaromatic polycyclic system D

m is an integer selected from 1 to 4. m is an integer being 1, 2, 3, or 4. m is preferably 3 or 4. If m is 1, one thiophene ring of a benzotrithiophene core according to any one of formulae (Ia), (Ib) and (Ic) is substituted by at one heteroaromatic polycyclic system D.
If m is 2, 3 or 4, many combinations of thiophene substitutions by heteroaromatic polycyclic system D are possible. Thus, if m is 2, either one thiophene ring of said core is substituted by two heteroaromatic polycyclic systems D or two thiophene rings of said core are substituted by at least one heteroaromatic polycyclic systems D.
If m is 3, either two thiophene rings of said core are substituted by at least one heteroaromatic polycyclic systems D (e.g. one ring is substituted by one system and the second ring by two systems) or at least each thiophene ring of said core is substituted by one heteroaromatic polycyclic systems D. At least each thiophene ring of a benzotrithiophene core according to any one of formulae (Ia), (Ib) and (Ic) is substituted by at least one heteroaromatic polycyclic system D.
If m is 4, either at least one thiophene ring of a benzotrithiophen core according to any one of formulae (Ia), (Ib) and (Ic) is substituted by at least one heteroaromatic polycyclic system D, one ring even being substituted twice, or two rings of said core are substituted twice by said heteroaromatic polyclcyclic systems D.

The dotted line in formula (1) represents the bond by which, if Ar_{y} is present (n being 1 or 2), Ar_{y} or, if Ar_{y} is absent (n being 0), Aₓ (o is 1) or, if Ar_{y} and Aₓ are absent (n and o are 0), Ar is connected to the thiophene ring of the BTT core.

R is selected from H, R₁, -NR₁R₂, -O-R₁, -P(=O) R₁R₂, -S-R₁, or halogen, wherein R₁ and R₂ are independently selected from C4-C20 aryl, C4-C20 heteroaryl, C4-C20 aryloxy group, C4-C20 heteroaryloxy group, C4-C20 alkoxyaryl, C4-C20 alkoxyheteroaryl, C4-C20 aryl aryloxy group, C4-C20 heteroaryl aryloxy group, C1-C20 alkyl, C1-C20 alkoxy group, C1-C20 alkoxyalkyl, C1-C20 alkylthio, C2-C20 alkenyl and C2-C20 alkynyl, wherein said alkyl, alkoxy, alkoxyalkyl, alkenyl and alkynyl, if they comprise 3 or more carbons, may be linear, branched or cyclic and wherein aryl, heteroaryl, alkyl, alkenyl, alkynyl may be further substituted by alkoxy group, alkylthio group and alkyl. Preferably R is selected from H, R₁ or -O-R₁.

R₁ and R₂ are independently selected from C4-C10 aryl, C4-C10 heteroaryl, C4-C10 aryloxy group, C4-C10 heteroaryloxy group, C4-C10 alkoxyaryl, C4-C10 alkoxyheteroaryl, C4-C10 aryl aryloxy group, C4-C10 heteroaryl aryloxy group, C1-C10 alkyl, C1-C10 alkoxy group, C1-C10 alkoxyalkyl, C1-C10 alkylthio, C2-C10 alkenyl and C2-C10 alkynyl, wherein said alkyl, alkoxy, alkoxyalkyl, alkenyl and alkynyl, if they comprise 3 or more carbons, may be linear, branched or cyclic and wherein aryl, heteroaryl, alkyl, alkenyl, alkynyl may be further substituted by alkoxy group, alkylthio group and alkyl. Preferably R₁ and R₂ are independently selected from C4-C10 aryl, C4-C10 heteroaryl, C1-C10 alkyl, C1-C10 alkoxy group, C1-C10 alkoxyalkyl, C1-C10 alkylthio, C2-C10 alkenyl and C2-C10 alkynyl.

Heteroatoms of the substituents R, R₁ and R₂ are selected from O, N, or S.

According to one embodiment, the substituents R of the polycyclic system D of formula (1) are identical.

Preferably, if Aₓ is N, n is 0 or 1, p is 1 or 2 and o is 1. If A_{X} is P(=O), preferably n is 1, p is 1 or 2 and o is 1.

Ar_{y} and Ar moieties may be identical or different. If A_{X} is N, n is 0 or 1, p is 1 or 2, o is 1, Ar_{y} moieties (if present) and Ar (if present) are identical or different, preferably identical. If A_{X} is P(=O), preferably n is 1, p is 1 or 2, Ar_{y} moieties and Ar moieties are identical or different, preferably identical.

In one embodiment, A_{X} is N and o is 1.

In a further embodiment, the integer o of the polycyclic sytem D of formula (1) is 1.

In one embodiment, Ar_{y} and Ar moieties of the polycyclic system D of formula (1) are independently selected from a moiety according to any one of formulae (2) to (28) 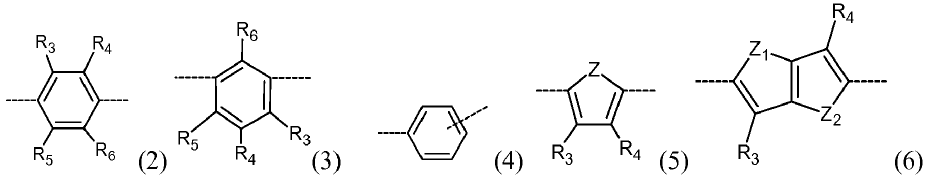 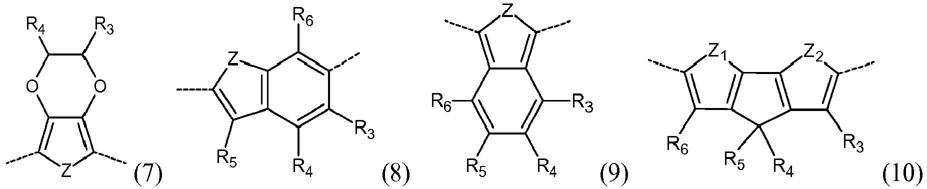 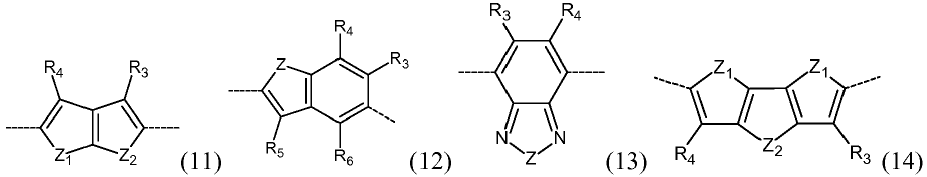 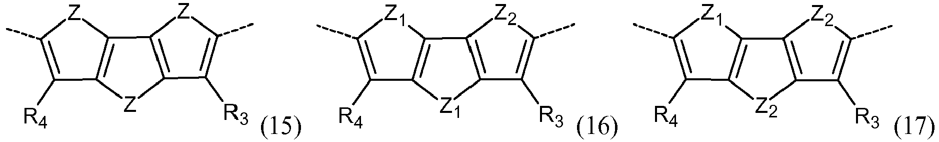 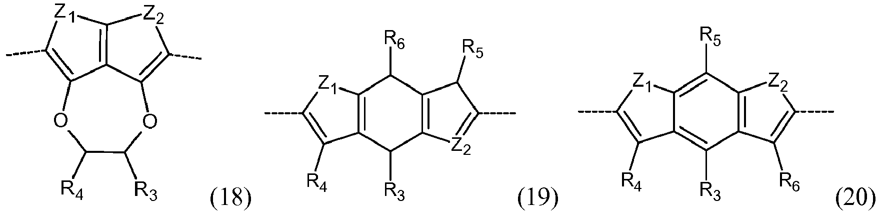 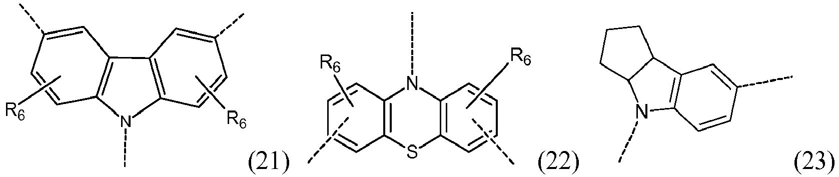 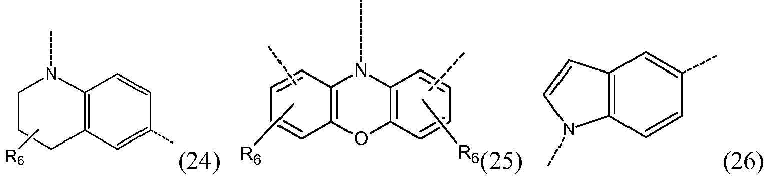 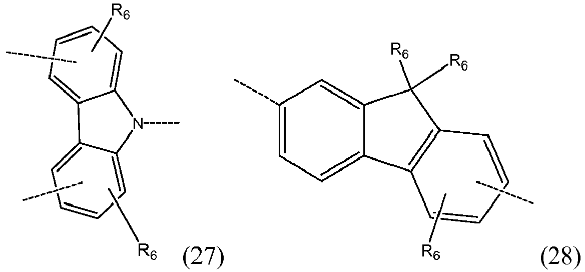 wherein
Z, Z₁, Z₂ are independently selected from O, S and Se atoms, and
R₃, R₄, R₅ and R₆ are independently selected from H, halogen (F, Cl, Br, I), C1-C10 alkyl, C1-C10 alkoxy group, C1-C10 alkylthio group (-S-alkyl) and -C≡N.

The dotted line in the moieties of formulae (2) to (28) represent the bond by which, the substituents are connected to the thiophene ring of the BTT core and/or to another substituents (R, e.g.) and/or to Aₓ.

Preferably, if present, Z, Z₁, Z₂ of the moieties of formulae (2) to 28) are independently selected from O and S.

If present in the same moiety, Z, Z₁ and Z₂ are preferably different from each other. In particular Z₁ and Z₂ are different from each other, if present in the same moiety.

In an embodiment R₃, R₄, R₅ and R₆ of moieties according to any one of formulae (2) to (28) are H.

In a further embodiment, Ar_{y} and Ar moieties of the polycyclic system D of formula (1) are independently selected from a moiety according to any one of formulae (2) to (4) and (21) to (27). If Ar_{y} and Ar moieties are different, Aₓ being N and o being 1, Ar_{y} moiety may be selected from a moiety according to any one of formulae (2) to (4) and (21) to (27) and Ar moiety may be selected from a moiety according to any one of formulae (2) to (4).

In another embodiment, the compound of formula (I) of the optoelectronic and/or photelectrochemical device of the invention is selected from a compound according to any one of formulae (Ia-1), (Ib-1) and (Ic-1) 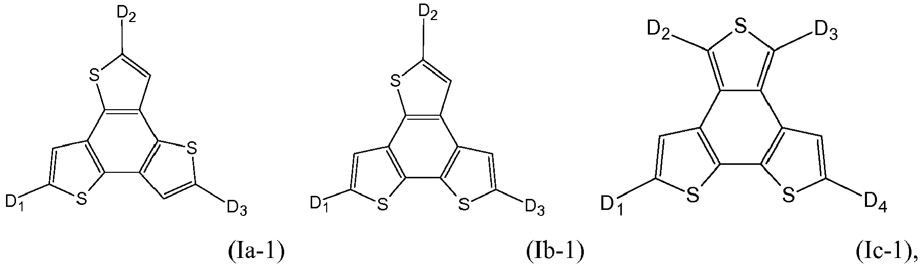 wherein
D₁, D₂, D₃, and D₄ are independently selected from a polycyclic system D of formula (1). D₁, D₂, D₃, and D₄ are of formula (1). The polycyclic systems D substituting the same BTT core Q of a compound of formula (I) are identical or different, preferably identical. The polycyclic systems D substituting the core of formula (Ia) or of formula (Ib) or of the formula (Ic) are preferably identical. Similarly, D₁, D₂, D₃ of the compound of formula (Ia-1) or D₁, D₂, D₃ of formula (Ib-1) or D₁, D₂, D₃, D₄ of the compound of formula (Ic-1) may be different or identical, preferably identical.

In particular, the compound of formula (I) may be selected from a compound according to any one of formulae (29) to (33)

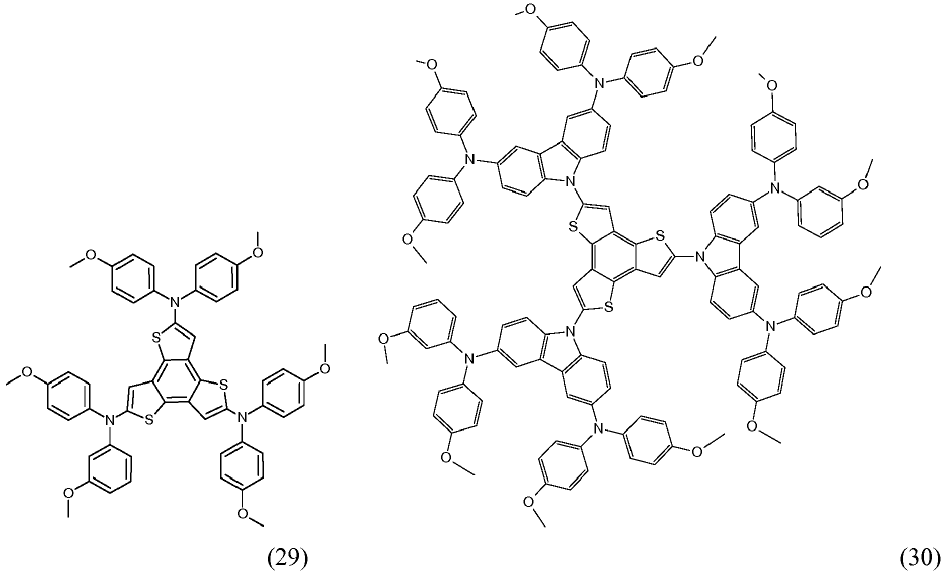

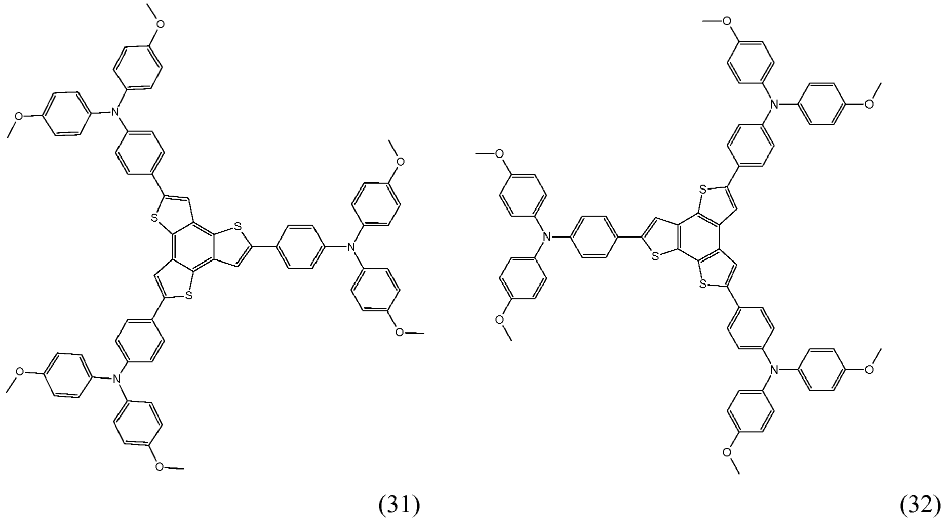

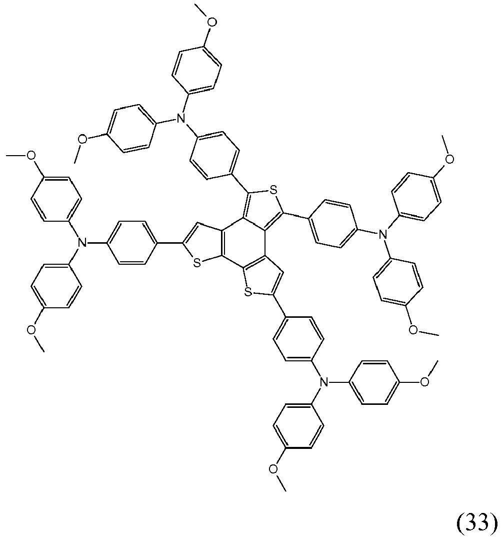

The optoelectronic and/or photoelectrochemical device of the invention comprises at least one compound according to any one of formulae (I), (Ia-1), (Ib-1) and (Ic-1) as defined above. In particular, the optoelectronic and/or photoelectrochemical device of the invention comprises at least one organic-inorganic perovskite and at least one compound according to any one of formulae (I), (Ia-1), (Ib-1) and (Ic-1) as defined above.

In one embodiment, the optoelectronic and/or photoelectrochemical device of the invention comprises a hole transporting material, wherein said hole transporting material comprises at least one compound of formula (I). Said hole transporting material may comprise at least one compound according to any one of formulae (I), (Ia-1), (Ib-1) and (Ic-1) as defined above.

The invention also provides hole transporting material for optoelectronic and/or photoelectrochemical device comprising at least one compound according to any one of formulae (I), (Ia-1), (Ib-1) and/or (Ic-1).

The compound of the invention may function as a hole transporting material and as a hole injection material to bring holes extracted from a sensitizer or absorber to the hole collector of the optoelectronic and/or photoelectrochemical device, a photovoltaic device, e.g. a solid solar cell. This compound is able to passivate the sensitizer or absorber layer and to improve the performance and the efficiency of such a device, and in particular an optoelectronic and/or photoelectrochemical device comprising an organic-inorganic perovskite as absorber.

By "hole transport material", "hole transporting material", "charge transporting material", "organic hole transport material" and "inorganic hole transport material", and the like, is meant any material or composition wherein charges are transported by electronic motion across said material or composition. The "hole transport material" is thus an electrically conductive material. Such hole transport materials, etc., are different from electrolytes. In this latter, charges are transported by diffusion over charge transporting material.

In another embodiment, the optoelectronic and/or photoelectrochemical device of the invention is selected from an organic photovoltaic device, a photovoltaic solid state device, a p-n heterojunction, an organic solar cell, a solid state solar cell, a phototransistor, photodetector, particle detector and OLED (organic light-emitting diode).

In a further embodiment, the optoelectronic and/or photoelectrochemical device of the invention is selected from a photovoltaic solid state device, a solid state solar cell or a solar cell.

The optoelectronic and/or photoelectrochemical device of the invention also comprises a conducting support layer, n-type semiconductor, a light-harvester layer or a sensitizer layer, a hole transporting layer and a counter electrode and/or metal layer. The optoelectronic and/or photoelectrochemical device may comprise an optional surface-increasing scaffold structure. Said metal layer may be doped as well as the n-type semiconductor. A conductive layer comprising a conductive material may be present between the hole transporting layer and the counter electrode and/or metal layer. The hole transporting layer may be provided on the sensitizer layer and is between the sensitizer layer and the conducting current providing layer, if present, or the counter electrode and/or metal layer. Further layer may be present.

According to a further embodiment, the optoelectronic and/or photoelectrochemical device of the invention may comprise a combination of two or more compounds of the invention according to any one of formulae (I), (Ia-1), (Ib-1) and (Ic-1) as defined above. Said compounds may be used as hole transporting material. The hole transporting material may be under the form of a layer and may comprise the combination of two or more compounds of the invention according to any one of formulae (I), (Ia-1), (Ib-1) and (Ic-1) as defined above.

The optoelectronic and/or photoelectrochemical device of the invention may comprise a hole collector layer, a conductive layer, an electron blocking layer, a sensitizer layer and a current collector layer, wherein the hole collector layer is coated by the conductive layer; wherein the electron blocking layer is between the conductive layer and the sensitizer layer, which is in contact with the current collector layer being a metal or a conductor. The hole collector layer comprises a hole transporting material comprising at least one compound of the invention according to any one of formulae (I), (Ia-1), (Ib-1) and (Ic-1) as defined above.

The conductive material is selected from one or more conductive polymers or one or more hole transporting materials, which may be selected from poly(3,4-ethylenedioxythiophene):poly(styrenesulfonate) (PEDOT:PSS), poly(3,4-ethylenedioxythiophene):poly(styrenesulfonate): grapheme nanocomposite (PEDOT:PSS:graphene), poly(N-vinylcarbazole) (PVK) and sulfonated poly(diphenylamine) (SPDPA), preferably from PEDOT:PSS, PEDOT:PSS:graphene and PVK, more preferably from PEDOT:PSS. Conductive polymers may also be selected from polymers comprising polyaniline, polypyrrole, polythiophene, polybenzene, polyethylenedioxythiophene, polypropylenedioxy-thiophene, polyacetylene, and combinations of two or more of the aforementioned, for example.

The conducting support layer is preferably substantially transparent. "Transparent" means transparent to at least a part, preferably a major part of the visible light. Preferably, the conducting support layer is substantially transparent to all wavelengths or types of visible light. Furthermore, the conducting support layer may be transparent to non-visible light, such as UV and IR radiation, for example.

The conducting support layer provides the support layer of photovoltaic solid state device. Preferably, the optoelectronic and/or photoelectrochemical device is built on said support layer. The support of the device may be also provided on the side of the counter electrode. In this case, the conductive support layer does not necessarily provide the support of the device, but may simply be or comprise a current collector, for example a metal foil.

The conducting support layer preferably functions and/or comprises a current collector, collecting the current obtained from the device. The conducting support layer may comprise a material selected from indium doped tin oxide (ITO), fluorine doped tinoxide (FTO), ZnO-Ga₂O₃, ZnO-Al₂O, tin-oxide, antimony doped tin oxide (ATO), SrGeO₃ and zinc oxide, preferably coated on a transparent substrate, such as plastic or glass. In this case, the plastic or glass provides the support structure of the layer and the cited conducting material provides the conductivity. Such support layers are generally known as conductive glass and conductive plastic, respectively, which are thus preferred conducting support layers in accordance with the invention. The conducting support layer comprises a conducting transparent layer, which may be selected from conducting glass and from conducting plastic.

The surface-increasing scaffold structure is provided on said conducting support structure or on a protective layer that may be provided on said scaffold structure. The surface-increasing scaffold structure is nanostructured and/or mesoporous.

The scaffold structure is made from and/or comprises a metal oxide. For example, the material of the scaffold structure is selected from semiconducting materials, such as Si, TiO₂, SnO₂, ZrO₂, Al₂O₃, Fe₂O₃, ZnO, WO₃, Nb₂O₅, CdS, ZnS, PbS, Bi₂S₃, CdSe, CdTe, SrTiO₃, GaP, InP, GaAs, CuInS₂, CuInSe₂, and combinations thereof, for example. Preferred semiconductor materials are Si, TiO₂, SnO₂, ZnO, WO₃, Nb₂O₅ and SrTiO₃, for example. According to an embodiment, the surface-increasing scaffold structure is nanostructured and/or nanoporous.

The invention does not intend to exclude the possibility that there are one or more intermediate layers between the scaffold structure and the conductive support. Such intermediate layers, if present, would preferably be conducting and/or semiconducting.

The sensitizer layer of the optoelectronic and/or photoelectrochemical device comprises at least one pigment being selected from organic, inorganic, organometallic and organic-inorganic pigments or a combination thereof. The sensitizer is preferably a light absorbing compound or material. Preferably, the sensitizer is a pigment, and most preferably the sensitizer is an organic-inorganic pigment.

The sensitizer layer or light-harvester layer may comprise one or more pigments of the group consisting of organometallic sensitizing compounds (phthalocyanine derived compounds, porphyrine derived compounds), metal free organic sensitizing compounds (diketopyrrolopyrrole (DPP) based sensitizer), inorganic sensitizing compounds such as quantum dots, Sb₂S₃ (Antimonysulfide, for example in the form of thin films), aggregates of organic pigments, nanocomposites, in particular organic-inorganic perovskites, and combinations of the aforementioned.

In an embodiment, the optoelectronic and/or photoelectrochemical device is selected from a photovoltaic solid state device or a solar cell comprising at least one inorganic perovskite or one organic-inorganic perovskite as sensitizer in addition to the at least one compound of the invention according to any one of formulae (I), (Ia-1), (Ib-1) and (Ic-1) as defined above. In a preferred embodiment, the sensitizer is an organic-inorganic perovskite.

Further the optoelectronic and/or photoelectrochemical device of the invention is selected from a photovoltaic solid state device or a solar cell comprising an organic-inorganic perovskite as sensitizer under the form of a layer.

The light-harvester layer or the sensitizer layer comprises, consists of or is made of an organic-inorganic perovskite. Said organic-inorganic perovskite is provided under a film of one perovskite pigment or mixed perovskite pigments or perovskite pigments mixed with further dyes or sensitizers.

The sensitizer layer may comprise a further pigment in addition to the organic-inorganic perovskite pigment, said further pigment selected from organic pigment, organometallic pigment or inorganic pigment.

The term "perovskite", for the purpose of this specification, refers to the "perovskite structure" and not specifically to the perovskite material, CaTiO₃. For the purpose of this specification, "perovskite" encompasses and preferably relates to any material that has the same type of crystal structure as calcium titanium oxide and of materials in which the bivalent cation is replaced by two separate monovalent cations. The perovskite structure has the general stoichiometry WMX₃, where "W" and "M" are cations and "X" is an anion. The "W" and "M" cations can have a variety of charges and in the original Perovskite mineral (CaTiO₃), the W cation is divalent and the M cation is tetravalent. For the purpose of this invention, the perovskite formulae includes structures having three (3) or four (4) anions, which may be the same or different, and/or one or two (2) organic cations, and/or metal atoms carrying two or three positive charges, in accordance with the formulae presented elsewhere in this specification.

The optoelectronic and/or photoelectrochemical device of the invention may comprise one or more layers of at least one organic-inorganic perovskite. In said device, the last upper layer of organic-inorganic perovskite is coated by the hole transporting layer comprising a hole transporting material as defined above, preferably comprising at least one compound according to any one of formulae (I), (Ia-1), (Ib-1) and (Ic-1) as defined above

In an embodiment, the optoelectronic and/or photoelectrochemical device of the invention, wherein the organic-inorganic perovskite layer material comprises a perovskite-structure according any one of formulae (II), (IIa), (IIb), (IIc), (IId) and (IIe) below:

WW'MX₄ (II)

WMX₃ (IIa)

WW'N_{2/3}X₄ (IIb)

WN_{2/3}X₃ (IIc)

BN_{2/3}X₄ (IId)

BMX₄ (IIe),

wherein
W and W' are organic, monovalent cations that are independently selected from primary, secondary, tertiary or quaternary organic ammonium compounds, including N-containing heterorings and ring systems, W and W' having independently from 1 to 60 carbons and 1 to 20 heteroatoms;
B is an organic, bivalent cation selected from primary, secondary, tertiary or quaternary organic ammonium compounds having from 1 to 60 carbons and 2-20 heteroatoms and having two positively charged nitrogen atoms;
M is a divalent metal cation selected from the group consisting of Cu²⁺, Ni²⁺, Co²⁺, Fe²⁺, Mn²⁺, Cr²⁺, Pd²⁺, Cd²⁺, Ge²⁺, Sn²⁺, Pb²⁺, Eu²⁺, or Yb²⁺;
N is selected from the group of Bi³⁺ and Sb³⁺; and,
X is independently selected from Cl-, Br⁻, I⁻, NCS⁻, CN⁻, and NCO⁻.

In particular, the three or four X may be identical or different. For example, in WMX₃ (formula IIa) may be expressed as formula (IIa'):

WMXiXiiXiii (IIa'),

wherein Xi, Xii, Xiii are independently selected from Cl-, Br⁻, I⁻, NCS⁻, CN⁻, and NCO⁻, preferably from halides (Cl⁻, Br⁻, I⁻), and W and M are as defined elsewhere in this specification. Xi, Xii, Xiii may thus be the same or different in this case.

Preferably, if Xi, Xii, Xiii in formulae (IIa) and (IIc) or Xi, Xii, Xiii, Xiv in formulae (II), (IIb), (IId) or (IIe) comprise different anions X, there are not more than two different anions. For example, Xi and Xii being the same with Xiii being an anion that is different from Xi and Xii.

According to a preferred embodiment, said organic-inorganic perovskite layer comprises a perovskite-structure according to any one of the formulae (IIf) to (III):

WPbX₃ (IIf)

WSnX₃ (IIg)

WBiX₄ (IIh)

WW'PbX₄ (IIi)

WW'SnX₄ (IIj)

BPbX₄ (IIk)

BSnx₄ (IIl)

wherein W, W', B and X are as defined above in this specification. Preferably, X is preferably selected from Cl-, Br⁻ and I⁻, most preferably X is I⁻ or a mixture of Br⁻ and I⁻.

According to a preferred embodiment, said organic-inorganic perovskite layer comprises a perovskite-structure of the formulae (IIf) to (IIl), more preferably (IIf) and/or (IIg) above.

According to an embodiment, W and W' are monovalent cations selected independently from any one of the compounds of formulae (34) to (42) below: 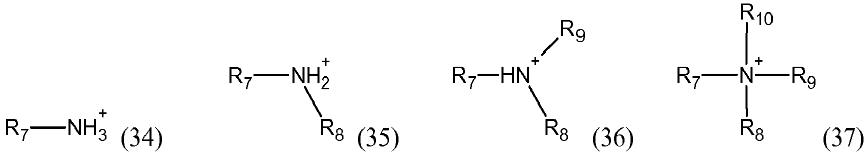 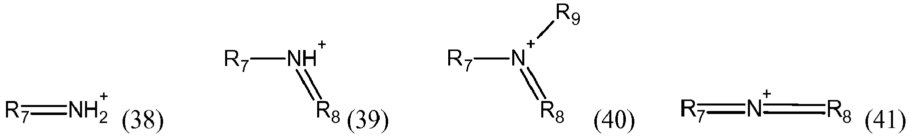 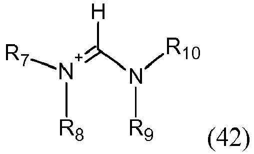 wherein,
R₇, R₈, R₉ and R₁₀ is independently selected from H or C1-C15 organic substituents comprising from 0 to 15 heteroatoms.

According to an embodiment, any one, several or all hydrogens in said C1-C15 organic substituents may be replaced by halogen and said organic substituent may comprise up to fifteen (15) N, S or O heteroatoms, and wherein, in any one of the compounds (20) to (28), the two or more of substituents present (R₇, R₈, R₉ and R₁₀, as applicable) may be covalently connected to each other to form a substituted or unsubstituted ring or ring system. Preferably, in a chain of atoms of said C1-C15 organic substituent, any heteroatom is connected to at least one carbon atom. Preferably, neighboring heteroatoms are absent and/or heteroatom-heteroatom bonds are absent in said C1-C15 organic substituent comprising from 0 to 15 heteroatoms. The heteroatoms may be selected from N, S, and/or O.

According to an embodiment, R₇, R₈, R₉ and R₁₀ are independently selected from C1 to C15 aliphatic and C4 to C15 aromatic or heteroaromatic substituents, wherein any one, several or all hydrogens in said substituent may be replaced by halogen and wherein, in any one of the compounds (34) to (42), the two or more of the substituents present may be covalently connected to each other to form a substituted or unsubstituted ring or ring system. According to a preferred embodiment, the organic-inorganic perovskite in the device of the invention is selected from a compound of formula (II), (IIa), (IIf) or (IIi).

According to an embodiment, B is a bivalent cation selected from any one of the compounds of formulae (43) and (44) below: 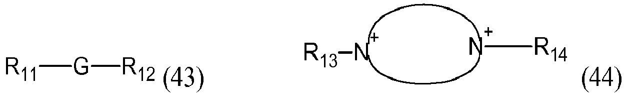 wherein,
in the compound of formula (43), G is an organic linker structure having 1 to 10 carbons and 0 to 5 heteroatoms selected from N, S, and/or O, wherein one or more hydrogen atoms in said G may be replaced by halogen;
wherein R₁₁ and R₁₂ are independently selected from a compounds of any one of formulae (34) to (42); and wherein, in the compound of formula (44), the circle containing said two positively charged nitrogen atoms represents a substituted or unsubstituted aromatic ring or ring system comprising 4 to 15 carbon atoms and 2 to 7 heteroatoms or 4 to 10 carbon atoms and 2 to 5 heteroatoms, wherein said nitrogen atoms are ring heteroatoms of said ring or ring system, and wherein the remaining of said heteroatoms may be selected independently from N, O and S and wherein R₁₃ and R₁₄ are independently selected from H and from a compounds of any one of formulae (34) to (42). Halogen atom substituting hydrogen atom totally or partially may also be present in addition to and/or independently of said 2 to 7 heteroatoms.

Preferably, if the number of carbons is in G is impair, the number of heteroatoms is smaller than the number of carbons. Preferably, in the ring structure of formula (44), the number of ring heteroatoms is smaller than the number of carbon atoms. According to an embodiment, G is an aliphatic, aromatic or heteroaromatic linker structure having from 1 to 10 carbons.

According to an embodiment, R₇, R₈, R₉ and R₁₀ are independently selected from C1 to C10 alkyl, C2 to C10 alkenyl, C2 to C10 alkynyl, C4 to C10 heteroaryl and C6 to C10 aryl, wherein said alkyl, alkenyl, and alkynyl, if they comprise 3 or more carbons, may be linear, branched or cyclic, wherein said heteroaryl and aryl may be substituted or unsubstituted, and wherein several or all hydrogens in R₇, R₈, R₉ and R₁₀ may be replaced by halogen. According to an embodiment, R₇, R₈, R₉ and R₁₀ are independently selected from C1 to C8 alkyl, C2 to C8 alkenyl, C2 to C8 alkynyl, C4 to C8 heteroaryl and C6 to C8 aryl, wherein said alkyl, alkenyl, and alkynyl, if they comprise 3 or more carbons, may be linear, branched or cyclic, wherein said heteroaryl and aryl may be substituted or unsubstituted, and wherein several or all hydrogens in R₇, R₈, R₉ and R₁₀ may be replaced by halogen.

According to an embodiment, R₇, R₈, R₉ and R₁₀ are independently selected from C1 to C6 alkyl, C2 to C6 alkenyl, C2 to C6 alkynyl, C4 to C6 heteroaryl and C6 aryl, wherein said alkyl, alkenyl, and alkynyl, if they comprise 3 or more carbons, may be linear, branched or cyclic, wherein said heteroaryl and aryl may be substituted or unsubstituted, and wherein several or all hydrogens in R₇, R₈, R₉ and R₁₀ may be replaced by halogen.

According to an embodiment, R₇, R₈, R₉ and R₁₀ are independently selected from C1 to C4 alkyl, C2 to C4 alkenyl and C2 to C4 alkynyl, wherein said alkyl, alkenyl and alkynyl, if they comprise 3 or more carbons, may be linear, branched or cyclic, and wherein several or all hydrogens in R₇, R₈, R₉ and R₁₀ may be replaced by halogen.

According to an embodiment, R₇, R₈, R₉ and R₁₀ are independently selected from C1 to C3, preferably C1 to C2 alkyl, C2 to C3, preferably C2 alkenyl and C2 to C3, preferably C2 alkynyl, wherein said alkyl, alkenyl and alkynyl, if they comprise 3 or more carbons, may be linear, branched or cyclic, and wherein several or all hydrogens in R₇, R₈, R₉ and R₁₀ may be replaced by halogen.

According to an embodiment, R₇, R₈, R₉ and R₁₀ is independently selected from C1 to C4, more preferably C1 to C3 and even more preferably C1 to C2 alkyl. Most preferably R₇, R₈, R₉ and R₁₀ are methyl. Again, said alkyl may be completely or partially halogenated.

According to an embodiment, W, W' and B are monovalent (W, W') and bivalent (B) cations, respectively, selected from substituted and unsubstituted C5 to C6 rings comprising one, two or more nitrogen heteroatoms, wherein one (for W and W') or two (for B) of said nitrogen atoms is/are positively charged. Substituents of such rings may be selected from halogen and from C1 to C4 alkyls, C2 to C4 alkenyls and C2 to C4 alkynyls as defined above, preferably from C1 to C3 alkyls, C3 alkenyls and C3 alkynyls as defined above. Said ring may comprise further heteroatoms, which may be selected from O, N and S. Bivalent organic cations B comprising two positively charged ring N-atoms are exemplified, for example, by the compound of formula (30) above. Such rings may be aromatic or aliphatic.

W, W' and B may also comprise a ring system comprising two or more rings, at least one of which being from substituted and unsubstituted C5 to C6 ring as defined as above. The elliptically drawn circle in the compound of formulae (30) may also represent a ring system comprising, for example, two or more rings, but preferably two rings. Also if W and/or W' comprises two rings, further ring heteroatoms may be present, which are preferably not charged, for example.

According to an embodiment, however, the organic cations W, W' and B comprise one (for W, W'), two (for B) or more nitrogen atom(s) but are free of any O or S or any other heteroatom, with the exception of halogens, which may substitute one or more hydrogen atoms in cation W and/or B.

W and W' preferably comprise one positively charged nitrogen atom. B preferably comprises two positively charged nitrogen atoms.

W, W' and B may be selected from the exemplary rings or ring systems of formulae (45) and (46) (for W, W') and from (47) to (49) (for B) below: 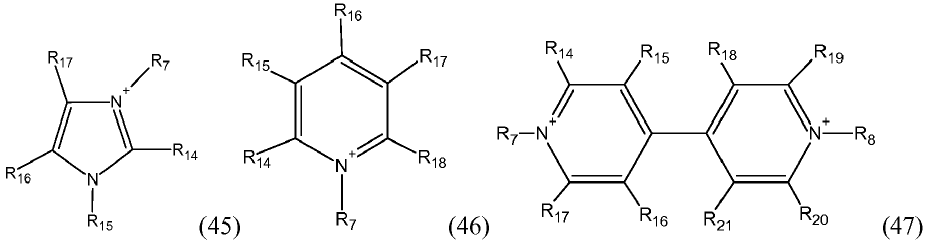 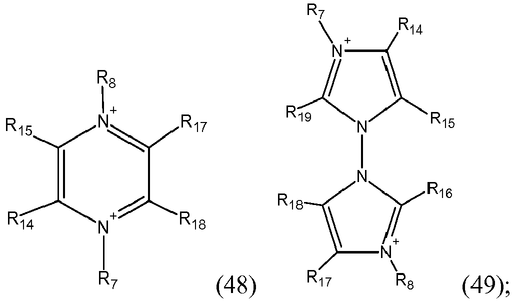 wherein
R₇ and R₈ are selected from substituents as defined above, and R₁₄, R₁₅, R₁₆₅ R₁₇, R₁₈, R₁₉, R₂₀ and R₂₁ are independently selected from H, halogen and substituents as defined above for R₇, R₈, R₉ and R₁₀. Preferably, R₁₄, R₁₅, R₁₆, R₁₇, R₁₈, R₁₉, R₂₀ and R₂₁ are selected from H and halogen, most preferably H.

In the organic cations W, W' and B, hydrogen atoms may be substituted by halogens, such as F, Cl, I, and Br, preferably F or Cl. Such a substitution is expected to reduce the hygroscopic properties of the perovskite layer or layers and may thus provide a useful option for the purpose of the present specification.

According to a preferred embodiment, W and W' are independently selected from organic cations of formula (34) and/or formula (44).

According to a preferred embodiment, the metal M is selected from Sn²⁺ and Pb²⁺, preferably Pb²⁺. According to a preferred embodiment, N is Sb³⁺.

According to a preferred embodiment, the three or four X are independently selected from Cl⁻, Br⁻, and I⁻.

In a further aspect, the invention also provides a p-n heterojunction comprising at least one organic-inorganic perovskite as sensitizer as defined above and at least one compound according to any one of formulae (I), (Ia-1), (Ib-1) and (Ic-1) as defined above.

In another aspect, the invention also provides a use of the compound of the invention according to any one of formulae (I), (Ia-1), (Ib-1) and (Ic-1) as a tuner of HOMO level.

The present invention will now be illustrated by way of examples. These examples do not limit the scope of this invention, which is defined by the appended claims.

### Examples

### Example 1: Synthesis of the compounds of formulae (29) to (33)

BTT central core was synthesized by a stepwise approach to obtain the tribromo-BTT, which involves the iodation of the 1,3,5-trichlorobenzene, Sonogashira cross-coupling reaction and ring closing reaction with sodium sulfide in NMP. In a subsequent synthetic step, bromination of the BTT employing NBS leads to the formation of the desired tribromo-derivative. Finally, *p*-methoxydiphenylamine or *p*-methoxydiphenylamine-substituted carbazole were introduced by Buchwald-Hartwig amination reaction to obtain tri-substituted BTT derivatives BTT-1 (compound of formula (29)) and BTT-2 (compound of formula (30)). On the other hand, *p*-methoxytriphenylamine units were covalently linked to the BTT core by a Suzuki cross-coupling reaction, affording BTT-3 (compound of formula (31)).

### Synthesis of tribromo-benzotrithiophene (tribromo-BTT)

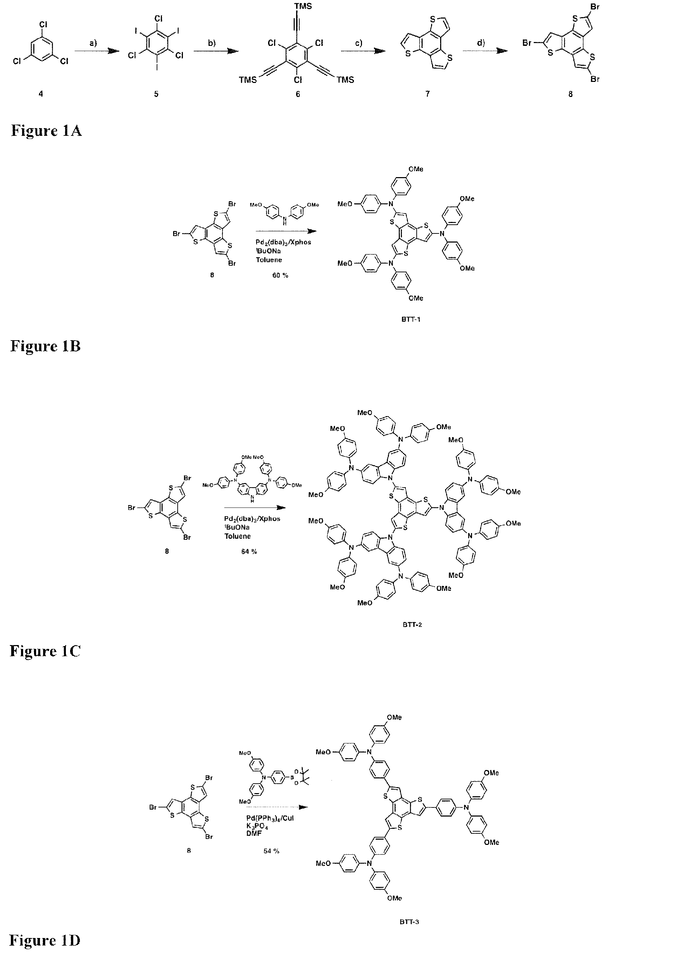

Bis(4-methoxyphenyl)amine, N3,N3,N6,N6-tetrakis(4-methoxyphenyl)-9H-carbazole-3,6-diamine, 4-(4,4,5,5-Tetramethyl-1,3,2-dioxaborolan-2-yl)-N,N-bis(4 methoxyphenyl)aniline, compounds 4, 5, 6, 7 of Figure 1A and benzotrithiophene (BTT) (compound 8 of Figure 1A) were prepared according to synthetic procedures known in the art.

### Synthesis of compound of formula (29) or BTT-1

Tribromo-BTT (150 mg, 0.31 mmol), bis(4-methoxyphenyl)amine (250 mg, 1.08 mmol), Pd₂(dba)₃ (20 mg, 0.02 mmol) and Xphos (15 mg, 0.03 mmol) were disolved in 21 mL of Toluene. The mixture was degassed for 30 minutes at room temperature. After that, sodium ter-butoxide (180 mg, 1.86 mmmol) was added and the reaction was heated at reflux for 3.5 hours under nitrogen atmosphere. After cooling to room temperature, the mixture was quenched by adding water and extracted with toluene. The combined organic extracts were dried over Na₂SO₄. After removal the solvent under reduced pressure the crude product was purified by flash column chromatography (silica gel, CH₂Cl₂) to afford BTT-1 (Figure 1B) as a yellow solid (174 mg, 0.19 mmol), yield 60 %.¹H NMR (400 MHz, CDCl₃) δ 7.16 (d, J = 8.9 Hz, 12H), 6.83 (d, J = 8.9 Hz, 12H), 6.53 (s, 3H), 3.79 (s, 18); ¹³C NMR (101 MHz, CDCl₃) δ 156.4, 153.0, 140.9, 130.6, 125.5, 121.6, 114.6, 107.6, 55.5; FTIR (neat): 3039, 2905, 2833, 1502, 1239, 1033, 866 cm⁻¹; HRMS calcd for C₅₄H₄₅N₃O₆S₃ [M⁺], 927.2465; found 927.2446.

### Synthesis of compound of formula (30) or BTT-2

Tribromo-BTT (300 mg, 0.62 mmol), N3,N3,N6,N6-tetrakis(4-methoxyphenyl)-9H-carbazole-3,6-diamine (1.27 g, 2.04 mmol), Pd₂(dba)₃ (39 mg, 0.04 mmol) and Xphos (29 mg, 0.06 mmol) were disolved in toluene (41 mL). The mixture was purged with nitrogen for 30 minutes at room temperature. After that, sodium ter-butoxide (358 mg, 3.72 mmmol) was added. The reaction was heated at reflux for 4 hours under nitrogen atmosphere. The mixture was quenched by adding water and extracted with toluene. The combined organic extracts were dried over Na₂SO₄. After removal the solvent under reduced pressure the crude product was purified by flash column chromatography (silica gel, CH₂Cl₂ and then CH₂Cl₂:AcOEt (120:1)) to afford BTT-2 (Figure 1C) as a yellow solid (842 mg, 0.40 mmol), yield 64 %.¹H NMR (400 MHz, THF-d₈) δ 7.99 (s, 3H), 7.69 (d, *J* = 2.1 Hz, 6H), 7.55 (d, *J* = 8.8 Hz, 6H), 7.15 (dd, *J* = 8.8, *J* = 2.1 Hz, 6H), 6.95 (d, *J* = 8.8 Hz, 24H), 6.76 (d, *J* = 8.8 Hz, 24H), 3.71 (s, 36H); ¹³C NMR (101 MHz, CDCl₃) δ 154.9, 142.3, 139.2, 138.1, 129.8, 129.4, 124.7, 124.6, 124.3, 118.6, 116.0, 114.6, 111.0, 55.5; FTIR (neat): 3040, 2939, 2831, 1501, 1452, 1234, 1033, 819 cm⁻¹; HRMS calcd for C₁₃₂H₁₀₅N₉O₁₂S₃ [M⁺], 2103.7039; found 2103.7085.

### Synthesis of compound of formula (31) or BTT-3

A solution of tribromo-BTT (300 mg, 0.62 mmol), 4-(4,4,5,5-Tetramethyl-1,3,2-dioxaborolan-2-yl)-N,N-bis(4-methoxyphenyl)aniline (1.07 gr, 2.48 mmol), K₃PO₄ (3.16 gr, 14.90 mmol) and Pd(PPh₃)₄ (179.4 mg, 0.15 mmol) in DMF (22 mL) was degassed for 1 hour under nitrogen. The reaction was heated at 80 °C for 18 hours. The mixture was cooled to room temperature and washed with NH₄Cl. The combined organic extracts (CH₂Cl₂)were dried over Na₂SO₄. After removal the solvent under reduced pressure the crude product was purified by flash column chromatography (silica gel, CH₂Cl₂ and then CH₂Cl₂:AcOEt (120:1)) to afford BTT-3 (Figure 1C) as a yellow solid (382 mg, 0.33 mmol), yield 54 %.¹H NMR (400 MHz, THF-d₈) δ 7.82 (s, 3H), 7.68 (d, *J* = 8.7 Hz, 6H), 7.12 (d, *J* = 8.7 Hz, 12H), 6.98 (d, *J =* 8.7 Hz, 6H), 6.91 (d, *J* = 8.7 Hz, 12H), 3.81 (s, 18H); ¹³C NMR (101 MHz, CDCl₃) δ 156.1, 148.8, 143.6, 140.5, 132.5, 129.7, 126.9, 126.8, 126.0, 120.2, 116.0, 114.8, 55.5; FTIR (neat): 3035, 2945, 2832, 1499, 1239, 1034, 821 cm⁻¹; HRMS calcd for C₇₂H₅₇N₃O₆S₃ [M⁺], 1155.3404; found 1155.3374.

### Example 2: Optoelectronic and/or electrochemical devices of the invention

### Device fabrication

Solar cell devices were fabricated onto FTO coated glass substrates. The substrates were cut and cleaned by sequential treatment with a 2% aqueous Helmanex solution and isopropanol in an ultrasonic bath for 30 and 5 minutes respectively followed by a 10 minutes UV-ozone exposure. The titania blocking layer was applied by spray pyrolysis from a precursor solution of titanium diisopropoxide bis(acetylacetonate) and acetonacetonate in ethanol at 450 °C in ambient atmosphere. A 150 nm thick mesoporous TiO₂ lay was obtained by spin coating a 30 nm titanium dioxide particle paste from Dyesol diluted (150mg / ml) in ethanol. The substrates were then sintered at 450 °C for 30 min under a dry air flow. The perovskite layer was applied by spin coating a precursor solution consisting of 1.25 M methylammonium iodide and 1.4 M lead iodide in dimethyl sulfoxide for MAPbI₃ and a solution of 1.4 M lead iodide, 0.17 M lead bromide, 0.15M methylammonium bromide and 1.08 M formamidinium iodide in a 4:1 mixture of dimethylformamide and dimethyl sulfoxide for the compositional modification (MAPbl₃)_{0.15}(MAPbl₃)_{0.85} respectively. The spin coating sequence includes two steps, the first one is at 1500 rpm for 10 s with a ramp of 200 rpm s⁻¹, and the second one is at 4000 rpm for 30s with a ramp of 2000 rpm s⁻¹. 10s prior to the end of the second step 120 µl of chlorobenzene are poured onto the spinning substrate. At the end of the spin program the substrate is immediately transferred onto a hotplate. The perovskite layer was kept at 100 °C for 1.5h in nitrogen atmosphere. The hole-transporting materials were dynamically spin-coated from solutions at 4000 rpm for 15 s. The hole-transporters were dissolved in chlorobenzene with concentrations of 0.06M for spiro-OMeTAD, 0.01M for BTT-1 (compound of formula (29)) and 0.03M for BTT-2 (compound of formula (30)), BTT-3 (compound of formula (31)), bbb-BTT (compound of formula (32)) as well as bbc-BTT (compound of formula (33)). Tert-butylpyridine (Tbp), bis(trifluoromethanesulfonyl)imide and Tris(2-(1H-pyrazol-1-yl)-4-tert-butylpyridine)cobalt(III) (FK209) and Tris(bis(trifluoromethylsulfonyl)imide)(Li-TFSI) were added as additives. Equimolar amounts of additives were added for all hole-transporters: 330 mol% Tbp, 50 mol% Li-TFSI from a 1.8M stock solution in acetonitrile and 3 mol% FK209 from a 0.25M stock solution in acetonitrile. For the electrodes 100 nm of gold was thermally evaporated under high vacuum.

### Solar cell characterization

High resolution cross-section images of the finished devices were recorded with a ZEISS Merlin HR-SEM scanning electron microscope. Current-voltage characteristics were measured in air under AM 1.5 simulated sunlight with a potentiostat (Keithley). The light intensity was measured for calibration with an NREL certified KG5 filtered Si reference diode. The solar cells were masked with a metal aperture of 0.16 cm² to define the active area. The current-voltage curves were recorded scanning at 10 mV s⁻¹.

### Example 3: Photovoltaic performances of optoelectronic and/or electrochemical devices of the invention with BTT-1, BTT-2, and BTT-3 compounds

### Photovoltaic performances with compounds BTT-1, BTT-2 and BTT-3 together with spiro-OMeTAD as a reference

Compounds BTT-1, BTT-2 and BTT-3 together with spiro-OMeTAD as a reference were tested on perovskite based solar cells. The cell architecture used in this study is the following: at the anode a compact and mesoporous layer of TiO₂ on top of the fluorine doped tin oxide (FTO) glass are responsible for the electron collection, while the cathode consists of a gold electrode thermally evaporated onto the HTM layer. For the photoactive layer, sandwiched in the middle, the most commonly used MAPbI₃ (methylammonium lead iodide) perovskite material is applied. In addition the performance was also tested on the compositional modification (FAPbI₃)_{0.85}(MAPbBr₃)_{0.15} (FAPbI₃: formamidinium lead iodide). Both perovskite layers were fabricated using a single step spin coating procedure. In short, the perovskite precursor solution was spin-coated at 4000 rpm for 30 s. Prior to the end of the spinning program 120 µl of chlorobenzene were poured onto the spinning substrate to obtain a homogeneous dense perovskite film after annealing for 1h at 100 °C. Compared to the standard perovskite, devices with the composite material have a thicker capping layer of around 400nm in respect to 150nm for the standard perovskite. Solutions of the doped HTM molecules were dynamically spin-coated on top of the perovskite layer at 4000 rpm for 15 s. The photovoltaic performance of the devices under AM 1.5 G (1 sun) conditions are shown in Figures 2A and 2B and the detailed results are summarized in the respective Tables 1 and 2 below.

**Table 1: photovoltaic performances of devices of the invention with MAPbI₃ perovskite material**

| Compounds | V_{oc} [mV] | J_{sc} [mA·cm⁻²] | FF [%] | PCE [%] |
|---|---|---|---|---|
| BTT-1 | 1043 | 20.44 | 72.3 | 15.99 |
| BTT-2 | 1092 | 20.59 | 76.7 | 16.98 |
| BTT-3 | 1065 | 21.90 | 76.7 | 18.17 |
| Spiro-OMeTAD | 1094 | 21.57 | 76.8 | 18.14 |

**Table 2: photovoltaic performances of devices of the invention with (FAPbI₃)_{0.85}(MAPbBr₃)_{0.15} perovskite material**

| Compounds | V_{oc} [mV] | J_{sc} [mA·cm⁻²] | FF [%] | PCE [%] |
|---|---|---|---|---|
| BTT-1 | 1039 | 21.63 | 70.6 | 15.96 |
| BTT-2 | 1027 | 22.33 | 74.7 | 17.54 |
| BTT-3 | 1037 | 22.65 | 72.4 | 17.28 |
| Spiro-OMeTAD | 1067 | 22.62 | 71.4 | 17.47 |

Current-voltage (IV) curves were recorded by applying a forward bias with a scan rate of 10 mVs⁻¹ in order to minimize hysteresis effects coming from the perovskite material. The molecule BTT-3 shows excellent performances and seems to work slightly better than its derivatives BTT-1 and BTT-2. Power conversion efficiencies (PCE) of up to 18.2 % were observed on the standard MAPbI₃ perovskite with only very little hysteresis. Up to date such high PCE values could only be achieved by spiro-OMeTAD or PTAA (Poly(triaryl amine)). Compared to BTT-1 and BTT-2 the main difference is the high current density of more than 21.9 mAcm⁻², which can be collected applying BTT-3 as an HTM. In fact those values are very close to what was obtained with the spiro-OMeTAD reference. Devices with BTT-2 on the other hand show a remarkably high open circuit voltage of almost 1.1 V, resulting in PCEs up to 17 %. For the devices prepared with the composite perovskite (FAPbI3)_{0.85}(MAPbBr3)_{0.15} the performances of the BTT HTM molecules are very similar. BTT-1 is the least effective HTM from the BTT-series due to a lower fill factor and lower current density. BTT-2 and BTT-3 are similar in performance and excellent PCE values of up to 17.5 % are obtained. In general the fill factor in all of the three molecules is excellent and comparable with spiro-OMeTAD, which suggests good carrier mobility in those materials.

### Electrical conductivity of compounds BTT-1, BTT-2 and BTT-3 together with spiro-OMeTAD as a reference

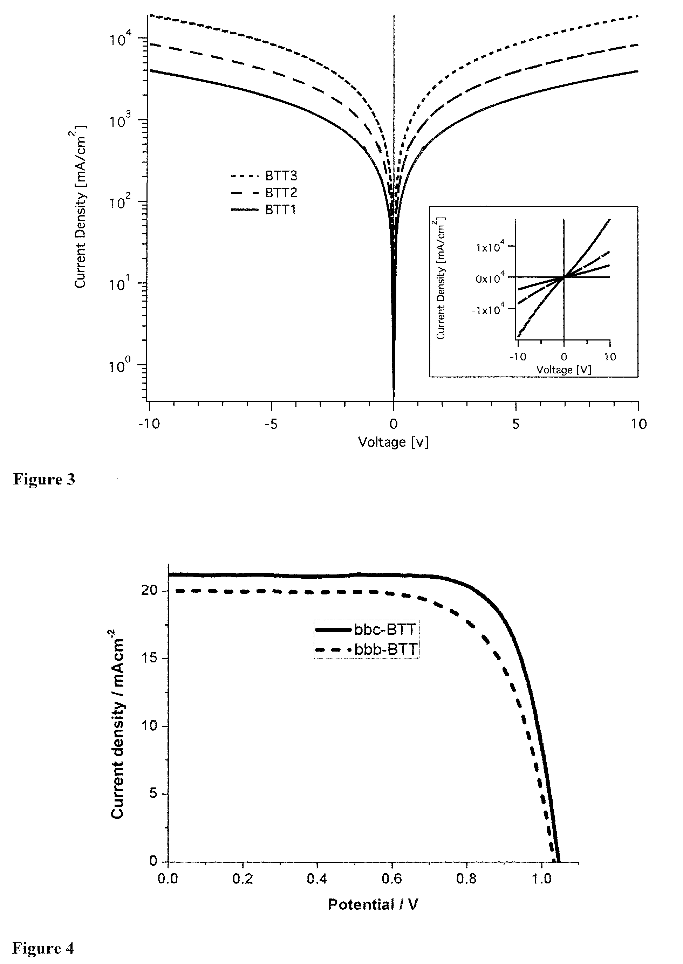

Electrical conductivity of the HTM molecules was measured in a lateral configuration between 2.5 µm spaced gold contacts. Thin films of the BTT molecules were spun onto OFET substrates using the same procedure as for the device making, adding 3 mole% of FK209 as a dopant. The results are displayed as a semi-logarithmic conductivity plot in Figure 3.

Conductivity values were extracted by fitting the curve to Ohms law. BTT-1 shows the lowest conductivity closely followed by BTT-2. The conductivity in BTT-3 is considerably higher. Although compared to spiro-OMeTAD the conductivity of BTT-3 is still about one order of magnitude lower, this value in combination with a thinner HTM layer seems to be enough for a good current collection. The trend obtained from the conductivity measurement clearly supports the observation in device performance of the three different BTT molecules. The conductivity of BTT-1 seems to be insufficient and very thin layers are needed, which increases the probability in generating shunting paths and thus loss in performance. The conductivity of BTT-2 is somewhat in the middle but high currents and therefore the best photovoltaic performance can be obtained with BTT-3, which we attribute to its enhanced conductivity.

In conclusion three new star-shaped small molecule hole-conducting materials BTT-1, BTT-2 and BTT-3 employed in perovskite solar cells show excellent performances. Energy conversion efficiencies of up to 16% for BTT-1 and up to 17% for BTT-2 were observed. Amazing power conversion efficiencies of up to 18.2 % were obtained with BTT-3 which is on the same level as spiro-OMeTAD used as a reference. To our knowledge this is one of the highest values observed for a small molecule HTM except for spiro-OMeTAD. The difference in performance between the three BTT molecules could be related to the conductivity measured in these HTM materials. The conductivity in BTT-1 and BTT-2 is considerably lower than the one found for BTT-3 thus limiting the amount of current that can be extracted from the device. Furthermore BTT-3 shows excellent band alignment with the MAPbI₃ perovskite. Based on excellent performances, hole-conductors based on the BTT core could be potential candidates to be used as a more cost-effective replacement to the nowadays widely used spiro-OMeTAD.

### Example 4: Photovoltaic performances of optoelectronic and/or electrochemical devices of the invention with bbb-BTT compound (compound of formula (32)) and bbc-BTT compound (compound of formula (33))

### Photovoltaic performances with compounds bbb-BTT and bbc-BTT

Compounds bbb-BTT and bbc-BTT were tested on perovskite based solar cells. The cell architecture used in this study is the following: at the anode a compact and mesoporous layer of TiO₂ on top of the fluorine doped tin oxide (FTO) glass are responsible for the electron collection, while the cathode consists of a gold electrode thermally evaporated onto the HTM layer. For the photoactive layer, sandwiched in the middle, the most commonly used MAPbI₃ (methylammonium lead iodide) perovskite material is applied. The perovskite layers were fabricated using a single step spin coating procedure. In short, the perovskite precursor solution was spin-coated at 4000 rpm for 30 s. Prior to the end of the spinning program 120 µl of chlorobenzene were poured onto the spinning substrate to obtain a homogeneous dense perovskite film after annealing for 1h at 100 °C. Solutions of the doped HTM molecules were dynamically spin-coated on top of the perovskite layer at 4000 rpm for 15 s. The photovoltaic performance of the devices under AM 1.5 G (1 sun) conditions are shown in Figure 4 and the detailed results are summarized in Table 3 below.

**Table 3: photovoltaic performances of devices of the invention with MAPbI₃ (Methylammonium lead iodide) perovskite material**

| Compounds | V_{oc} [mV] | J_{sc} [mA·cm⁻²] | FF [%] | PCE [%] |
|---|---|---|---|---|
| bbb-BTT | 1033 | 20.00 | 69.3 | 14.8 |
| bbc-BTT | 1056 | 21.19 | 75.0 | 17.3 |

## Claims

1. An optoelectronic and/or photoelectrochemical device comprising at least one organic-inorganic perovskite as sensitizer and at least one compound of formula (I) 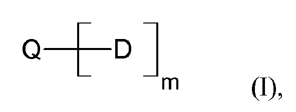 wherein
m is an integer selected from 1 to 4;
Q is a heteroaromatic polycyclic system comprising one benzotrithiophene core selected from a core according to any one of formulae (Ia), (Ib) and (Ic) 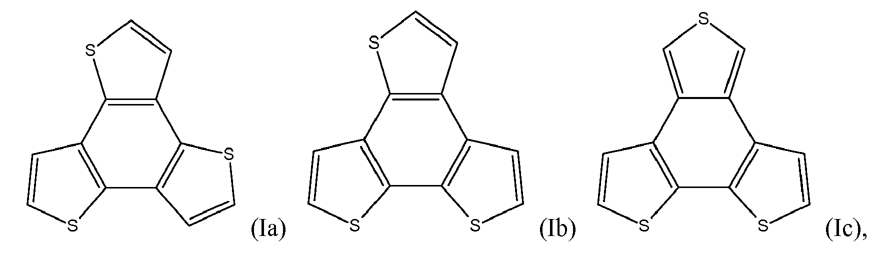 wherein
at least one thiophene ring of said core is substituted by at least one heteroaromatic polycyclic system D;
D is selected from a polycyclic system of formula (1) 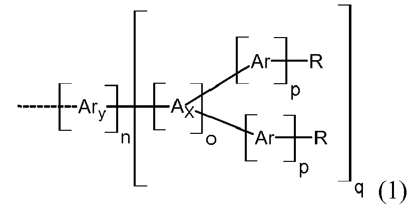 wherein,
q is an integer selected from 1 or 2;
o is an integer selected from 0 or 1;
n and p are integers independently selected from 0, 1 or 2 with the proviso that, if n and o are 0, p is 1 or 2;
A_{X} is selected from N or P(=O), preferably N;
Ar_{y} and Ar are independently selected from a monocyclic system or a polycyclic system comprising fused aromatic rings or conjugated monocyclic aromatic rings, said ring comprising 0, 1 or 2 heteroatoms being selected from O, S, Se and N, and is further substituted in addition to R by other substituents independently selected from H, halogen (F, Cl, Br, I), C1-C10 alkyl, C1-C10 alkoxy group, C1-C10 alkylthio (-S-alkyl) and -C≡N;
R is selected from H, R₁, -NR₁R₂, -O-R₁, -P(=O) R₁R₂, -S-R₁, or halogen (F, Cl, Br, I), wherein R₁ and R₂ are independently selected from C4-C20 aryl, C4-C20 heteroaryl, C4-C20 aryloxy group, C4-C20 heteroaryloxy group, C4-C20 alkoxyaryl, C4-C20 alkoxyheteroaryl, C4-C20 aryl aryloxy group, C4-C20 heteroaryl aryloxy group, C1-C20 alkyl, C1-C20 alkoxy group, C1-C20 alkoxyalkyl, C1-C20 alkylthio, C2-C20 alkenyl and C2-C20 alkynyl, wherein said alkyl, alkoxy, alkoxyalkyl, alkenyl and alkynyl, if they comprise 3 or more carbons, may be linear, branched or cyclic and wherein aryl, heteroaryl, alkyl, alkenyl, alkynyl may be further substituted by alkoxy group, alkylthio group and alkyl.

2. The optoelectronic and/or photoelectrochemical device according to claim 1, wherein m of the compound of formula (I) is 3 or 4.

3. The optoelectronic and/or photoelectrochemical device according to any one of the preceding claims, wherein the compound of formula (I) is selected from a compound according to any one of formulae (Ia-1), (Ib-1) and (Ic-1) 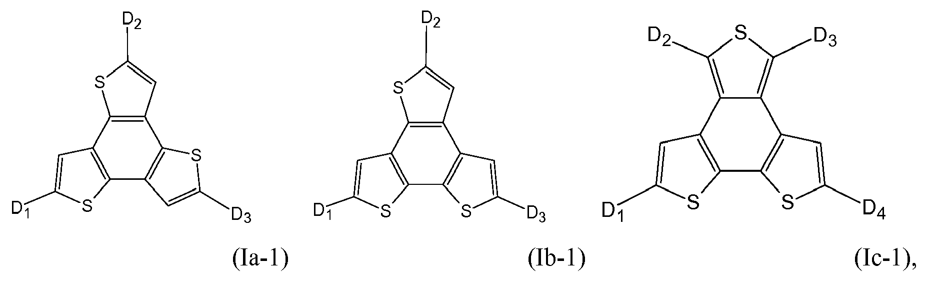 wherein
D₁, D₂, D₃, and D₄ are independently selected from a moiety of polycyclic systems D of formula (1).

4. The optoelectronic and/or photoelectrochemical according to any one of the preceding claims, wherein Ar_{y} and Ar of the polycyclic system D of formula (1) are independently selected from a moiety according to any one of formulae (2) to (28) 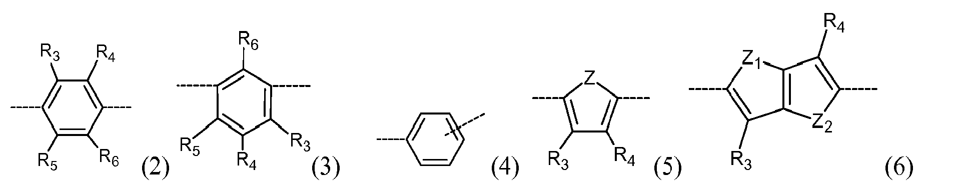 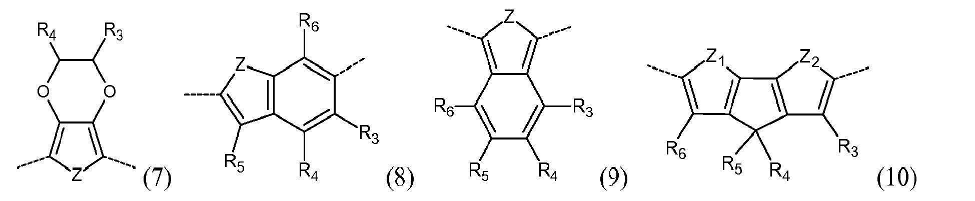 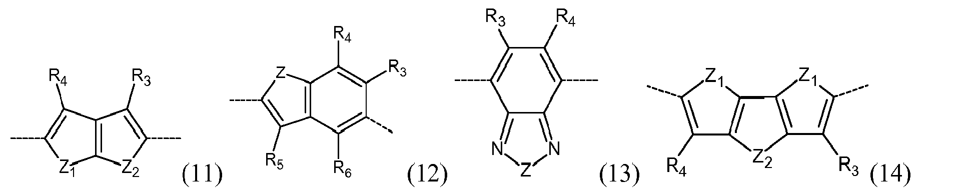 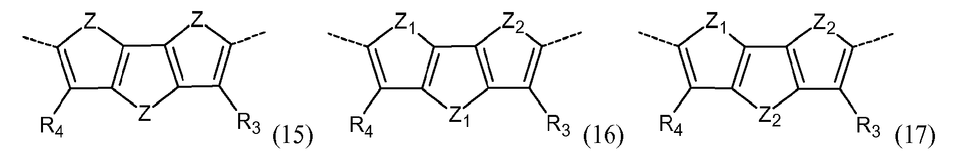 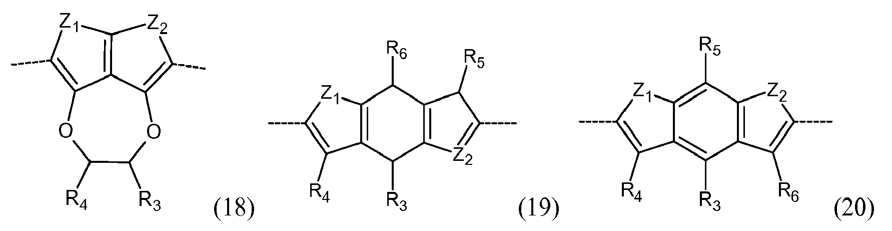 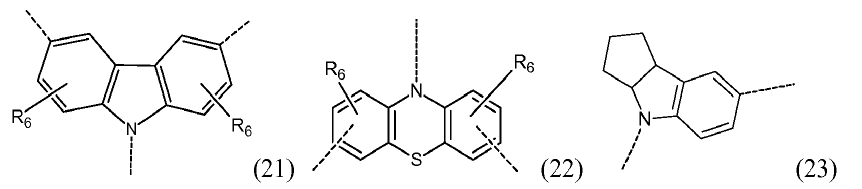 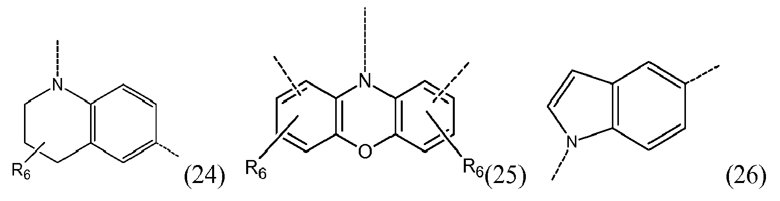 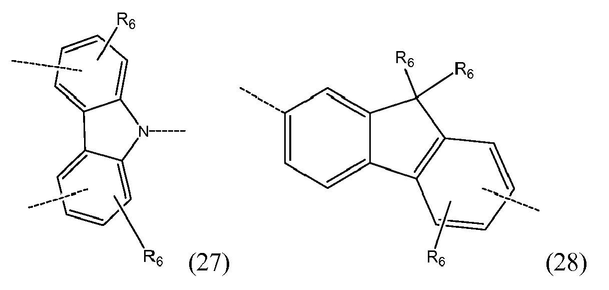 wherein
Z, Z₁, Z₂ are independently selected from O, S and Se atoms, and
R₃, R₄, R₅ and R₆ are independently selected from H, halogen (F, Cl, Br, I), C1-C10 alkyl, C1-C10 alkoxy group, C1-C10 alkylthio group (-S-alkyl) and -C≡N.

5. The optoelectronic and/or photoelectrochemical according to any one of the preceding claims, wherein the integer o of the polycyclic system D of formula (1) is 1.

6. The optoelectronic and/or photoelectrochemical according to any one of the preceding claims, wherein the substituents R of the polycyclic system D of formula (I) are identical

7. The optoelectronic and/or photoelectrochemical according to any one of the preceding claims, wherein the polycyclic systems D of the compound of formula (I) are identical.

8. The optoelectronic and/or photoelectrochemical according to any one of claims 3-6, wherein R₃, R₄, R₅ and R₆ of moieties according to any one of formulae (2) to (28) are H.

9. The optoelectronic and/or photoelectrochemical according to any one of the preceding claims, wherein Ar_{y} and Ar of the polycyclic system D of formula (1) are independently selected from a moiety according to any one of formulae (2) to (4) and (21) to (27).

10. The optoelectronic and/or photoelectrochemical device according to any one of the preceding claims comprising a hole transporting material, wherein said hole transporting material comprises at least one compound of formula (I).

11. The optoelectronic and/or photoelectrochemical device according to any one of the preceding claims, wherein the at least one organic-inorganic perovskite is selected from a perovskite-structure according any one of formulae (II), (IIa), (IIb), (IIc), (IId) and (IIe)
WW'MX₄ (II)
WMX₃ (IIa)
WW'N_{2/3}X₄ (IIb)
WN_{2/3}X₃ (IIc)
BN_{2/3}X₄ (IId)
BMX₄ (IIe),
wherein
W and W' are organic monovalent cations that are independently selected from primary, secondary, tertiary or quaternary organic ammonium compounds, including N-containing heterorings and ring systems, W and W' having independently from 1 to 60 carbons and 1 to 20 heteroatoms;
B is an organic, bivalent cation selected from primary, secondary, tertiary or quaternary organic ammonium compounds having from 1 to 60 carbons and 2-20 heteroatoms and having two positively charged nitrogen atoms;
M is a divalent metal cation selected from the group consisting of Cu²⁺, Ni²⁺, Co²⁺, Fe²⁺, Mn²⁺, Cr²⁺, Pd²⁺, Cd²⁺, Ge²⁺, Sn²⁺, Pb²⁺, Eu²⁺, or Yb²⁺;
N is selected from the group of Bi³⁺ and Sb³⁺; and,
X is independently selected from Cl-, Br⁻, I⁻, NCS⁻, CN⁻, and NCO⁻.

12. The optoelectronic and/or photoelectrochemical device according to any one of the preceding claims, wherein said device is selected from an organic photovoltaic device, a photovoltaic solid state device, a p-n heterojunction, an organic solar cell, a solid state solar cell, a phototransistor, photodetector, particle detector and OLED (organic light-emitting diode).

13. The optoelectronic and/or photoelectrochemical device according to any one of the preceding claims, wherein said device is selected from a photovoltaic solid state device, a solid state solar cell or a solar cell.

14. A p-n heterojunction comprising at least one organic-inorganic perovskite as sensitizer and at least one compound of formula (I) as defined in claims 1 to 9.

15. Use of a compound according to any one of claims 1 to 8 to modulate the HOMO level.
